(19) Europäisches Patentamt
European Patent Office
Office européen des brevets

(11) EP 2 506 952 B1

(12) # FASCICULE DE BREVET EUROPEEN

(45) Date de publication et mention de la délivrance du brevet:
**19.08.2015 Bulletin 2015/34**

(21) Numéro de dépôt: **10801598.3**

(22) Date de dépôt: **19.11.2010**

(51) Int Cl.:
***B01D 53/14*** *(2006.01)* ***B01D 53/34*** *(2006.01)*
***B01D 53/40*** *(2006.01)*

(86) Numéro de dépôt international:
**PCT/FR2010/000775**

(87) Numéro de publication internationale:
**WO 2011/064470 (03.06.2011 Gazette 2011/22)**

(54) **SOLUTION ABSORBANTE CONTENANT UN INHIBITEUR DE DÉGRADATION. DÉRIVÉ DE LA PYRIMIDINE OU DE LA TRIAZINE ET PROCÉDÉ D'ABSORPTION DE COMPOSÉS ACIDES CONTENUS DANS UN EFFLUENT GAZEUX**

ABSORPTIONSLÖSUNG MIT EINEM ABBAUHEMMER AUS PYRIMIDIN ODER TRIAZIN UND VERFAHREN ZUR ABSORPTION VON SÄUREVERBINDUNGEN IN EINEM ABGAS

ABSORBENT SOLUTION CONTAINING A BREAKDOWN INHIBITOR DERIVED FROM PYRIMIDINE OR FROM TRIAZINE AND ABSORPTION PROCESS FOR ACID COMPOUNDS CONTAINED IN A GASEOUS EFFLUENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.11.2009 FR 0905753**

(43) Date de publication de la demande:
**10.10.2012 Bulletin 2012/41**

(73) Titulaire: **IFP Energies nouvelles**
**92852 Rueil-Malmaison Cedex (FR)**

(72) Inventeurs:
- **DELFORT, Bruno**
  **F-75005 Paris (FR)**
- **CARRETTE, Pierre-Louis**
  **F-69005 Lyon (FR)**

(56) Documents cités:
**WO-A1-99/10561 WO-A2-2009/156619**
**US-A- 2 655 543 US-A1- 2005 202 967**

- **"Mechanical design and operation of ehtanolamine plants ED - Arthur L Kohl; Fred C Riesenfeld", 1 janvier 1979 (1979-01-01), GAS PURIFICATION, GULF PUBLISHING COMPANY, PAGE(S) 91 - 123, XP009111641, ISBN: 9780872013131 le document en entier**
- **BUTWELL K F ET AL: "Alkanolamine treating", HYDROCARBON PROCESSING, GULF PUBLISHING CO. HOUSTON, US, no. 3, 1 mars 1982 (1982-03-01), pages 108-116, XP009111638, ISSN: 0018-8190**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne le domaine de la désacidification d'un effluent gazeux. Plus précisément la présente invention propose des composés pour réduire la dégradation d'une solution absorbante mise en oeuvre pour absorber les composés acides contenus dans un effluent gazeux, la solution absorbante comportant des amines en solution aqueuse. En particulier, l'invention concerne des composés utilisés pour réduire la dégradation des amines utilisées pour la désacidification de gaz contenant de l'oxygène, comme par exemple les fumées de combustion.

La désacidification des effluents gazeux, tels que par exemple le gaz naturel et les fumées de combustion, est généralement réalisée par lavage par une solution absorbante. La solution absorbante permet d'absorber les composés acides présents dans l'effluent gazeux ($H_2S$, mercaptans, $CO_2$, COS, $SO_2$, $CS_2$).

La désacidification de ces effluents, notamment la décarbonatation et la désulfuration, impose des contraintes spécifiques à la solution absorbante, en particulier une stabilité thermique et chimique notamment face aux impuretés de l'effluent, à savoir essentiellement l'oxygène, les SOx et les NOx. L'oxygène peut aussi entrer en contact avec la solution absorbante sans être forcément présent dans l'effluent gazeux à traiter comme dans le cas par exemple d'une entrée accidentelle d'air au niveau des bacs de stockage de solution absorbante.

Les solutions absorbantes les plus utilisées aujourd'hui sont les solutions aqueuses d'alcanolamines. On peut citer le document FR 2 820 430 qui propose des procédés de désacidification d'effluents gazeux.

Toutefois, il est bien connu de l'homme de l'art que ces amines présentent l'inconvénient de se dégrader dans les conditions de mise en oeuvre.

En particulier, les amines peuvent être dégradées par l'oxygène engendrant une consommation de l'amine et la formation de produits de dégradation qui s'accumulent dans l'unité ou, pour les plus volatils, qui sont entraînés dans les effluents gazeux du procédé. Ainsi, en particulier dans le cas du traitement de fumées en post-combustion dans un procédé utilisant une solution aqueuse de monoéthanolamine (MEA) des quantités importantes d'ammoniac sont formées. L'ammoniac ainsi formé est entraîné dans l'atmosphère avec les fumées traitées ce qui pose des problèmes quant à la protection de l'environnement.

Dans le cas du captage du $CO_2$ dans les fumées issues d'unités industrielles ou de production d'électricité ou d'énergie en général, les phénomènes de dégradation de la solution absorbante aux amines sont accrus par la présence d'une quantité massive d'oxygène dans la charge à traiter pouvant aller jusqu'à 5% en volume en général. Dans le cas de fumées issues de cycle combiné au gaz naturel, la teneur volumique d'oxygène dans les fumées peut atteindre 15%.

La solution dégradée se caractérise par :

- une baisse de l'absorption des composés acides de la charge par rapport à une solution fraîche d'amine,
- une augmentation de la densité de la solution absorbante, ainsi que de sa viscosité, pouvant entraîner une perte de performance,
- la formation d'amines plus volatiles polluant le gaz traité et le gaz acide issu de l'étape de régénération : ammoniac, méthylamine, diméthylamine et triméthylamine par exemple selon la nature de l'amine utilisée,
- une accumulation de produits de dégradation dans la solution absorbante qui peut entraîner la nécessité d'un traitement de la solution dégradée,
- d'éventuels problèmes de moussage dus aux produits de dégradation.

La dégradation de la solution absorbante pénalise donc les performances et le bon fonctionnement des unités de désacidification des gaz.

Pour pallier le problème de dégradation, à défaut de pouvoir limiter ou supprimer la présence d'oxygène dans la solution absorbante, on ajoute, dans la solution absorbante, des composés dont le rôle est de prévenir ou limiter les phénomènes de dégradation des composés amines, notamment la dégradation engendrée par les phénomènes d'oxydation. Ces composés sont couramment nommés additifs inhibiteurs de dégradation. Les principaux modes d'action connus des additifs inhibiteurs de dégradation consistent selon leur nature en une réaction de type réduction et/ou en un captage, un piégeage et/ou une stabilisation des radicaux formés dans la solution absorbante afin de limiter ou d'empêcher ou d'interrompre les réactions, notamment les réactions en chaîne, de dégradation.

Le brevets US 5686016 cite des additifs utilisés pour limiter la dégradation de solutions absorbantes utilisées pour la désacidification du gaz naturel, en particulier les oximes.

Le brevet US 7056482 cite des additifs utilisés pour limiter la dégradation de solutions absorbantes utilisées pour le captage du $CO_2$, en particulier les thiosulfates et les sulfites.

La demande de brevet US 2005/0202967 décrit une solution absorbante pour désacidifier des effluents gazeux comportant un amine aliphatique et un antioxydant non-hydroquinoide, tel qu'un composé triazine.

De manière générale, la présente invention décrit une famille d'additifs inhibiteurs de dégradation qui permet notamment de réduire la dégradation d'une solution absorbante mise en oeuvre pour l'absorption de composés acides contenus dans un effluent gazeux, la solution absorbante comportant des composés amines en solution aqueuse.

La présente invention décrit un procédé pour absorber des composés acides contenus dans un effluent gazeux, dans lequel on met en contact l'effluent gazeux avec une solution absorbante, ladite solution comportant :

a) au moins une amine,
b) de l'eau,
c) au moins un composé inhibiteur de dégradation pour limiter la dégradation de ladite amine, ledit composé inhibiteur de dégradation répondant à l'une des formules générales suivantes :

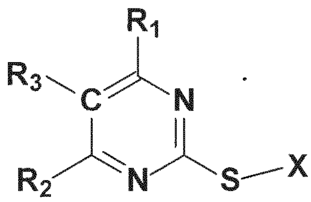

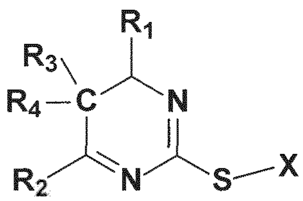

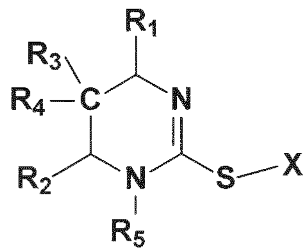

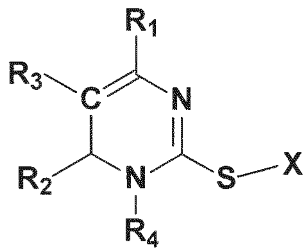

dans lesquelles X est choisi parmi

- un atome d'hydrogène,
- un élément alcalin ou alcalino-terreux,
- un métal monovalent ou multivalent,
- un cation ammonium $NH_4^+$ ou résultant de la protonation d'une fonction amine,
- un cation phosphonium,

dans lesquelles chacun des radicaux $R_1$, $R_2$ et $R_3$ est choisi indépendamment parmi :

- un atome d'hydrogène,
- un radical hydrocarboné comprenant 1 à 12 atomes de carbone,
- un radical

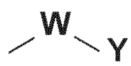

dans lequel W est choisi parmi un atome de soufre et un atome d'oxygène et Y est choisi parmi :

- un atome d'hydrogène,
- un radical hydrocarboné comprenant de 1 à 12 atomes de carbone,
- un élément alcalin ou alcalino-terreux,
- un métal monovalent ou multivalent,
- un cation ammonium $NH_4^+$ ou résultant de la protonation d'une fonction amine,
- un cation phosphonium,

et dans lesquelles chacun des radicaux $R_4$ et $R_5$ est choisi indépendamment parmi:
- un atome d'hydrogène,
- un radical hydrocarboné comprenant de 1 à 12 atomes de carbone.

Au moins l'un des radicaux $R_1$, $R_2$, $R_3$, X et Y peut être un groupe hydrocarboné renfermant entre 1 et 12 atomes de carbone et peut contenir, en outre, au moins un composé choisi parmi un hétéroatome et un halogène.

Au moins l'un des radicaux $R_1$, $R_2$, $R_3$, X et Y peut être un groupe hydrocarboné renfermant entre 1 et 12 atomes de carbone et peut comporter, en outre, au moins une fonction choisie parmi le groupe : une fonction hydroxyle, une fonction cétone, une fonction carboxylique et une fonction nitrile.

La solution peut comporter entre 10% et 99% poids d'amine, entre 1% et 90% poids d'eau et entre 5 ppm et 5% poids de composé inhibiteur de dégradation.

Le composé inhibiteur de dégradation peut être choisi parmi le groupe contenant : le 2-thiouracil (ou 4-hydroxy-2-mercaptopyrimidine), un sel du 2-thiouracil (ou 4-hydroxy-2-mercaptopyrimidine), le 4-thiouracil (ou 2-hydroxy-4-mercaptopyrimidine), un sel du 4-thiouracil (ou 2-hydroxy-4-mercaptopyrimidine), l'acide 2-thiobarbirurique (ou 4,6-dihydroxypyrimidine-2-thiol), un sel de l'acide 2-thiobarbirurique (ou 4,6-dihydroxypyrimidine-2-thiol), la 2-mercaptopyrimidine, un sel de la 2-mercaptopyrimidine, le 3,4,5,6-tétrahydropyrimidine-2-thiol, un sel du 3,4,5,6-tétrahydropyrimidine-2-thiol, le 4,5-dihydro-4,4,6-triméthyl-2-pyrimidinethiol, un sel du 4,5-dihydro-4,4,6-triméthyl-2-pyrimidinethiol, la 4-hydroxy-2-mercapto-6-méthylpyrimidine, un sel de la 4-hydroxy-2-mercapto-6-méthylpyrimidine, la 2-mercapto-4-méthylpyrimidine, un sel de la 2-mercapto-4-méthylpyrimidine, et la 4-méthyl-2-(méthylthio)pyrimidine.

L'amine peut être choisie parmi le groupe contenant : la N,N,N',N',N"-pentaméthyldiéthylènetriamine, la pipérazine, la monoéthanolamine, la diéthanolamine, la méthyldiéthanolamine, la diisopropanolamine, la diglycolamine, un sel de la glycine et un sel de la taurine.

Dans le cas où l'amine est la monoéthanolamine, le composé inhibiteur de dégradation est choisi parmi l'acide 2-thiobarbiturique, le 2-thiouracil, le 3,4,5,6-tétrahydro-2-pyrimidinethiol.

La solution absorbante selon l'invention peut comporter au moins 39% poids de monoéthanolamine.

Dans le procédé selon l'invention, la solution aqueuse peut être mise en oeuvre pour absorber des composés acides contenus dans l'un des effluents du groupe contenant le gaz naturel, les fumées de combustion, les gaz de synthèse, les gaz de raffinerie, les gaz obtenus en queue du procédé Claus, les gaz de fermentation de biomasse, les gaz de cimenterie et les fumées d'incinérateur.

L'effluent gazeux peut comporter au moins 500ppm volumique d'oxygène.

D'autres caractéristiques et avantages de l'invention seront mieux compris et apparaîtront clairement à la lecture de la description faite ci-après, en référence aux figures annexées dans lesquelles les figures 1 et 2 représentent la teneur en $NH_3$ dans le gaz traité par une solution absorbante en fonction de l'ajout ou non d'additifs inhibiteurs de dégradation dans la solution absorbante et en fonction de la teneur en amine dans la solution absorbante.

Afin de réduire la dégradation d'une solution absorbante, les inventeurs ont montré que la dégradation d'une solution absorbante comportant des composés organiques munis d'une fonction amine en solution aqueuse est sensiblement réduite en présence d'une faible quantité d'additifs inhibiteurs de dégradation décrits ci-après.

Les additifs inhibiteurs de dégradation utilisés dans le procédé selon l'invention sont des composés appartenant à la famille des dérivés de la pyrimidine dont au moins un substituant contient un atome de soufre.

Dans la présente description, on entend par pyrimidine les composés cycliques à 6 atomes dont le cycle comporte 4 atomes de carbone et 2 atomes d'azote en position 1 et 3, et 3 insaturations.

Dans la présente description, on entend par dérivés de la pyrimidine dont au moins un substituant contient un atome de soufre, que au moins un des atomes de carbone du cycle pyrimidine soit lié à un atome de soufre.

Les composés inhibiteurs de dégradation utilisés dans le procédé selon l'invention peuvent par exemple répondre à la formule générale suivante :

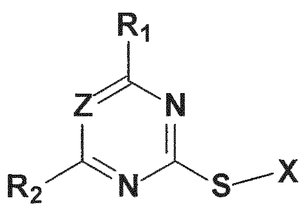

(formule I)

Z est tel que le motif

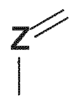

est :

◦ un motif

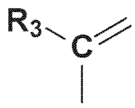

et dans ce cas la formule générale devient :

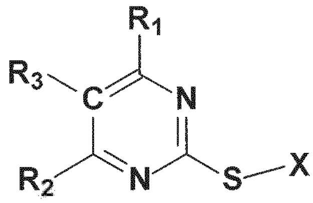
(formule III)

La formule III correspond à un dérivé de la pyrimidine dont au moins un substituant comporte un atome de soufre.

**Dans les formules I et III, X est choisi parmi:**

- un atome d'hydrogène,
- un élément alcalin ou alcalino-terreux,
- un métal monovalent ou multivalent,
- un cation ammonium $NH_4^+$ ou un cation résultant de la protonation d'une fonction amine,
- un cation phosphonium,
- un radical hydrocarboné comprenant 1 à 12 atomes de carbone, saturé ou non, linéaire, branché ou cyclique, hétérocyclique ou aromatique, pouvant éventuellement être relié à des hétéroatomes, des halogènes, pouvant renfermer des fonctions hydroxyles, cétones, carboxyliques ou nitriles.

Dans le cas où X est un élément multivalent, il est entendu que pour respecter la neutralité de la molécule, elle pourra prendre la forme

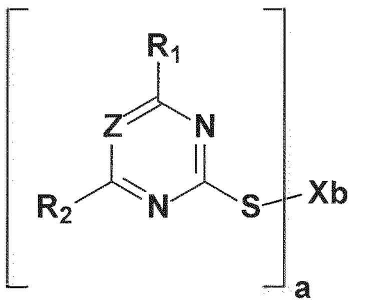

avec a et b étant des nombres entiers permettant le respect de la neutralité ou des valences dans le respect des règles de la chimie.

**Dans les formules I et III, R1, R2 et R3 sont indifféremment choisis parmi :**

- un atome d'hydrogène,
- un radical hydrocarboné comprenant 1 à 12 atomes de carbone, saturé ou non, linéaire, branché ou cyclique, hétérocyclique ou aromatique pouvant éventuellement être relié à des hétéroatomes, des halogènes, pouvant renfermer des fonctions hydroxyles, cétones, carboxyliques ou nitriles,
- un radical

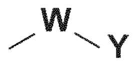

avec W étant un atome de soufre ou un atome d'oxygène et Y étant choisi parmi :

- ◦ un atome d'hydrogène,
- ◦ un radical hydrocarboné comprenant 1 à 12 atomes de carbone, saturé ou non, linéaire, branché ou cyclique, hétérocyclique ou aromatique, pouvant éventuellement être relié à des hétéroatomes, des halogènes, pouvant renfermer des fonctions hydroxyles, cétones, carboxyliques ou nitriles,
- ◦ un élément alcalin ou alcalino-terreux,
- ◦ un métal monovalent ou multivalent,
- ◦ un cation ammonium $NH_4^+$ ou résultant de la protonation d'une fonction amine,
- ◦ un cation phosphonium.

La formule générale III peut également s'étendre aux formes partiellement hydrogénées qui peuvent prendre les formes suivantes :

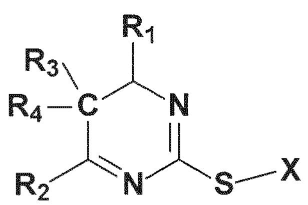

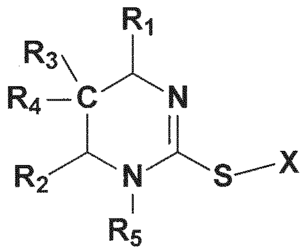

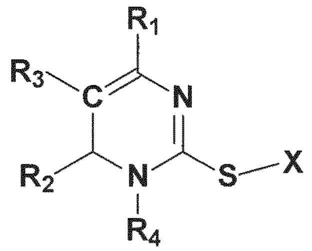

dans lesquelles $R_4$ et $R_5$ sont choisis parmi un atome d'hydrogène ou un radical comprenant 1 à 12 atomes de carbone, saturé ou non, linéaire, branché ou cyclique, hétérocyclique ou aromatique.

Les molécules utilisées dans le procédé de l'invention peuvent aussi exister sous leur forme dite tautomère lorsque cela est permis, et ce, dans le respect des règles de la chimie organique.

Les solutions absorbantes utilisées dans le procédé l'invention peuvent être mises en oeuvre pour désacidifier les effluents gazeux suivants : le gaz naturel, les gaz de synthèse, les fumées de combustion, les gaz de raffinerie, les gaz obtenus en queue du procédé Claus, les gaz de fermentation de biomasse, les gaz de cimenterie, les fumées d'incinérateur. Ces effluents gazeux contiennent un ou plusieurs des composés acides suivants : le $CO_2$, l'$H_2S$, des mercaptans, du COS, du $SO_2$, du $NO_2$, du $CS_2$. En particulier, le procédé selon l'invention, peut être mis en oeuvre pour absorber des composés acides contenus dans un effluent gazeux contenant de l'oxygène, comme par exemple les fumées de combustion. La teneur en oxygène dans l'effluent gazeux peut être supérieure à 500ppm en volume, de préférence supérieure à 0,5%, voire au moins 1%, 3% ou 5% volumique. En général, la teneur en oxygène dans l'effluent gazeux reste inférieure à 20% en volume. Les fumées de combustion sont produites notamment par la combustion d'hydrocarbures, de biogaz, de charbon dans une chaudière ou pour une turbine à gaz de combustion, par exemple dans le but de produire de l'électricité. Ces fumées peuvent comporter entre 50 % et 90 % d'azote, entre 5 % et 20 % de dioxyde de carbone. Les fumées comportent en général au moins 500ppm volumique, de préférence au moins 1% volumique, voire 2%, 3% ou 5% volumique d'oxygène, jusqu'à une teneur qui en général n'excède pas 20 % volume d'oxygène.

La mise en oeuvre d'une solution absorbante pour désacidifier un effluent gazeux est généralement réalisée en effectuant une étape d'absorption suivie d'une étape de régénération. L'étape d'absorption consiste à mettre en contact l'effluent gazeux avec la solution absorbante. Lors du contact, les composés organiques munis d'une fonction amine de la solution absorbante réagissent avec les composés acides contenus dans l'effluent de manière à obtenir un effluent gazeux appauvri en composés acides et une solution absorbante enrichie en composés acides. L'étape de régénération consiste notamment à chauffer et, éventuellement à détendre, au moins une partie de la solution absorbante enrichie en composés acides afin de libérer les composés acides sous forme gazeuse. La solution absorbante régénérée, c'est-à-dire appauvrie en composés acides est recyclée à l'étape d'absorption.

La solution absorbante utilisée dans le procédé selon l'invention comporte des composés organiques en solution aqueuse. De manière générale, les composés organiques sont des amines, c'est-à-dire qu'ils comportent au moins une fonction amine. Les composés organiques peuvent être en concentration variable par exemple compris entre 10% et 99% poids, de préférence entre 20% et 75% poids, voire entre 20% et 50% poids, dans la solution aqueuse. La solution

absorbante peut contenir entre 1% et 90% poids d'eau, de préférence entre 25% et 80% poids, voire entre 50% et 70% poids d'eau.

Par exemples les composés organiques sont des amines tels que la N,N,N',N',N''-pentaméthyldiéthylènetriamine ou la pipérazine. Par exemple la pipérazine est utilisée pour le traitement du gaz naturel et pour la décarbonatation des fumées de combustion.

Les composés organiques peuvent également être des alcanolamines telles que la monoéthanolamine (MEA), la diéthanolamine (DEA), la méthyldiéthanolamine (MDEA), la diisopropanolamine (DIPA) ou la diglycolamine. De préférence, la MDEA et la DEA sont couramment utilisées pour la désacidification du gaz naturel. La MEA est plus particulièrement utilisée pour la décarbonatation des fumées de combustion.

Les composés organiques peuvent également être des sels d'acides aminés tels que les sels de la glycine ou de la taurine qui sont notamment mis en oeuvre pour le captage du $CO_2$ dans les fumées de combustion.

En outre, la solution absorbante utilisée dans le procédé selon l'invention peut contenir des composés qui absorbent physiquement au moins partiellement un ou plusieurs composés acides de l'effluent gazeux. Par exemple la solution absorbante peut comporter entre 5% et 50% poids de composés absorbants à caractère physique tel que par exemple du méthanol, du sulfolane ou de la N-formyl morpholine.

Un autre avantage de l'invention réside dans le fait que l'utilisation d'additifs inhibiteurs de dégradation selon l'invention permet d'augmenter la concentration en amines communément utilisée par l'homme du métier et d'augmenter ainsi les performances du procédé : augmentation de la capacité et de la vitesse d'absorption des composés acides par la solution absorbante entraînant une réduction des coûts d'investissement et des coûts opératoires de l'unité industrielle. En effet, comme montré ci-après dans l'exemple 3, en absence d'additifs inhibiteurs de dégradation, la vitesse de dégradation des amines augmente avec l'augmentation de la concentration en amines. Ainsi dans le cas par exemple de l'utilisation d'une solution aqueuse de MEA (monoéthanolamine) pour le captage du $CO_2$ dans les fumées de combustion, la concentration en MEA est communément limitée à 30% poids pour limiter la dégradation de cette amine. Il est entendu ici que la concentration en amine est définie en pourcentage poids dans l'eau avant absorption de $CO_2$. Ainsi par exemple, une solution absorbante utilisée pour le captage du $CO_2$ dans une fumée de combustion et contenant un additif inhibiteur de dégradation selon l'invention peut contenir plus de 30% poids et de préférence plus de 35% poids de MEA, une bonne valeur de la concentration en MEA étant au moins égale à 39% poids.

Parmi l'ensemble des molécules appartenant à la famille des dérivés de la pyrimidine dont au moins un substituant contient un atome de soufre, on utilise de préférence les composés inhibiteurs de dégradation suivants :

le 2-thiouracil (ou 4-hydroxy-2-mercaptopyrimidine), le 4-thiouracil (ou 2-hydroxy-4-mercaptopyrimidine), l'acide 2-thiobarbirurique (ou 4,6-dihydroxypyrimidine-2-thiol), la 2-mercaptopyrimidine, le 3,4,5,6-tétrahydropyrimidine-2-thiol, le 4,5-dihydro-4,4,6-triméthyl-2-pyrimidinethiol, la 4-hydroxy-2-mercapto-6-méthylpyrimidine, la 2-mercapto-4-méthylpyrimidine, les sels des éléments précédemment cités, ainsi que la 4-méthyl-2-(méthylthio)pyrimidine.

Les sels des composés inhibiteurs de dégradation utilisés dans le procédé selon l'invention peuvent être obtenus par exemple par leur neutralisation à l'aide d'un hydroxyde ou un carbonate alcalin, alcalinoterreux ou métallique ou d'ammonium ou à l'aide d'une amine présente dans la solution absorbante. Dans ce cas, c'est au moins une fonction thiol qui sera neutralisée.

Les sels des composés inhibiteurs de dégradation utilisés dans le procédé selon l'invention peuvent être également obtenus par exemple par leur neutralisation à l'aide d'un acide organique ou inorganique. Dans ce cas, c'est au moins un atome d'azote présent dans le cycle qui sera protoné.

Les composés inhibiteurs de dégradation listés au paragraphe précédent sont particulièrement bien adaptés à la prévention de la dégradation d'amine en solution aqueuse mise en oeuvre dans un procédé de captage du $CO_2$ contenu dans des fumées de combustion.

Pour limiter la dégradation d'une solution absorbante composée d'amines, en particulier d'alcanolamines, par exemple la monoéthanolamine (MEA), en solution aqueuse notamment pour capter le $CO_2$ des fumées de combustion, on peut utiliser de préférence l'un des composés suivants :

l'acide 2-thiobarbiturique, le 2-thiouracil, le 3,4,5,6-tétrahydro-2-pyrimidinethiol ainsi que leurs sels tels par exemple les sels de sodium, de potassium ou d'ammonium.

De préférence, selon l'invention, on utilise l'acide 2-thiobarbiturique, le 2-thiouracil, le 3,4,5,6-tétrahydro-2-pyrimidinethiol pour limiter la dégradation d'une amine, en particulier la MEA, en solution aqueuse mise en oeuvre pour désacidifier un effluent gazeux, notamment dans le cadre du captage de $CO_2$ contenu dans des fumées de combustion.

La solution absorbante utilisée dans le procédé selon l'invention comporte une quantité d'additifs inhibiteurs de dégradation définis par la formule générale décrite ci-dessus. La solution absorbante peut comporter un ou plusieurs additifs inhibiteurs de dégradation différents correspondant à ladite formule générale. De plus, dans la solution absorbante, les additifs inhibiteurs de dégradation selon l'invention peuvent être associés à d'autres composés inhibiteurs de dégradation de familles chimiques différentes. Selon l'invention, la solution absorbante comporte entre 5 ppm et 5% poids d'additifs inhibiteurs de dégradation selon l'invention, de préférence de 50 ppm à 2% poids, et une excellente teneur en additifs inhibiteurs de dégradation dans la solution étant comprise entre 100 ppm et 1% poids.

Les exemples présentés ci-après permettent de comparer et d'illustrer les performances des additifs inhibiteurs de dégradation selon l'invention, en terme de réduction de la dégradation des amines en solution aqueuse, de réduction des émissions de composés de dégradation volatils et de possibilité d'augmenter la concentration en amines sans augmenter leur dégradation.

EXEMPLE 1 :

Les amines de la solution absorbante peuvent être dégradées dans une utilisation selon l'invention engendrant une consommation de l'amine.

Les essais de dégradation d'une amine en solution aqueuse sont effectués selon le mode opératoire suivant.

100g de solution de MEA (monoéthanolamine) 30% poids dans l'eau désionisée sont placés dans un réacteur en verre surmonté d'un condenseur pour éviter l'évaporation de l'eau. Le réacteur est chauffé à 80°C dans un bloc chauffant électrique. La solution est agitée à 1000 tours par minute par un barreau aimanté. La présence de contre pales empêche la formation d'un vortex. Un gaz est mis en contact avec la solution à l'aide d'un tube plongeant pendant 7 jours à pression atmosphérique. Selon les essais, on fait varier la nature du gaz mis en contact avec la solution. De même les essais sont conduits soit en absence soit en présence de différents additifs inhibiteurs de dégradation incorporés dans la solution aqueuse d'amine à 0,25% poids.

Lorsque l'essai est conduit uniquement en présence de $CO_2$ et en l'absence d'oxygène, le gaz mis en contact avec la solution est un mélange de 7Nl/h d'azote et de 0,033 Nl/h de $CO_2$ réalisé dans une chambre de mélange. Dans ce cas, le gaz comporte uniquement du $CO_2$ et de l'azote.

Lorsque l'essai est conduit en présence de $CO_2$ et d'oxygène, le gaz mis en contact avec la solution est un mélange de 7Nl/h d'air atmosphérique, c'est-à-dire de l'air ambiant non purifié, et de 0,033 Nl/h de $CO_2$ réalisé dans une chambre de mélange. Dans ce cas, le gaz contient du $CO_2$, de l'azote et de l'oxygène, la teneur en oxygène dans le gaz étant de 21% environ.

Une analyse par chromatographie en phase gazeuse de la solution ainsi dégradée est réalisée à la fin de l'essai. La méthode chromatographique utilise une colonne polaire, un gaz vecteur, l'hélium, un étalon interne, le triéthylèneglycol et une détection FID (Flame Induced Détection). Cette analyse permet de déterminer la concentration résiduelle de MEA et donc le taux de dégradation défini par :

$$taux_de_dégradation = \left(1 - \frac{[MEA]finale}{[MEA]initiale}\right) * 100$$

Le tableau 1 donne les taux de dégradation d'une solution aqueuse de MEA (monoéthanolamine) 30% poids, en présence d'un additif inhibiteur de dégradation ou non et soumise à un gaz renfermant de l'azote, du $CO_2$ et contenant ou non de l'oxygène :

- Cas n°1 : sans oxygène et sans additif
- Cas n°2 : en présence d'oxygène et sans additif
- Cas n°3 : en présence d'oxygène et en présence d'un additif anti-oxygène conventionnel, le sulfite de sodium ($Na_2SO_3$).
- Cas n°4 : en présence d'oxygène et en présence d'un additif selon l'invention, l'acide 2-thiobarbiturique.
- Cas n°5 : en présence d'oxygène et en présence d'un additif selon l'invention, le 2-thiouracil.
- Cas n°6 : en présence d'oxygène et en présence d'un additif selon l'invention, le 3,4,5,6-tétrahydro-2-pyrimidinethiol.
- Cas n°7 : en présence d'oxygène et en présence d'un additif, le trisel de sodium du 1,3,5-triazine-2,4,6-trithiol

Tableau 1 : comparaison des taux de dégradation de la MEA 30% poids obtenus dans l'eau à 80°C dans les différents cas.

| CAS | Teneur en $O_2$ | Nom de l'additif | Taux de dégradation |
|---|---|---|---|
| 1 | 0% | - | <3% |
| 2 | 21% | - | 70% |
| 3 | 21% | sulfite de sodium ($Na_2SO_3$) | 71% |

(suite)

| CAS | Teneur en $O_2$ | Nom de l'additif | Taux de dégradation |
|---|---|---|---|
| 4 | 21% | acide 2-thiobarbiturique | <3% |
| 5 | 21% | 2-thiouracil | <3% |
| 6 | 21% | 3,4,5,6-tétrahydro-2-pyrimidinethiol | <3% |
| 7 | 21% | trisel de sodium du 1,3,5-triazine-2,4,6-trithiol | <3% |

Il apparaît clairement que :

1. la solution de MEA n'est pas dégradée en présence du seul $CO_2$ en l'absence d'oxygène
2. la dégradation de la MEA est attribuable à la présence d'oxygène
3. en présence d'un additif anti-oxygène conventionnel tel le sulfite de sodium, la dégradation de la MEA en présence d'oxygène n'est pas diminuée
4. en présence d'additifs selon l'invention, la dégradation de la MEA est ramenée au même niveau que celle constatée en l'absence d'oxygène, c'est-à-dire considérée comme nulle car inférieure à l'incertitude de la mesure qui est de 3%.

En conclusion, les additifs selon l'invention combattent efficacement l'effet de l'oxygène sur la dégradation de la MEA.

EXEMPLE 2 :

En particulier, les amines peuvent être dégradées par l'oxygène engendrant la formation de produits volatils, qui sont entraînés dans les effluents gazeux du procédé. Ainsi, par exemple dans le cas du traitement de fumées en post-combustion dans un procédé utilisant une solution aqueuse de MEA des quantités importantes d'ammoniac sont formées. L'ammoniac ainsi formé est entraîné dans l'atmosphère avec les fumées traitées ce qui pose des problèmes quant à la protection de l'environnement.

La figure 1 présente un suivi de la concentration en ammoniac dans le gaz sortant du réacteur dans les cas 2, 3, 4, 5, 6 et 7 définis dans l'exemple 1. [A] correspond à la concentration en ammoniac en ppm volume dans le gaz de sortie du réacteur, t représente le temps exprimé en jours. Les cas 2, 3, 4, 5, 6 et 7 sont représentés respectivement par la courbe 2 en trait continu, la courbe 3 avec des carrés blancs, la courbe 4 avec des triangles blancs, la courbe 5 avec des croix, la courbe 6 avec des losanges blancs et la courbe 7 avec des ronds noirs.

La concentration en ammoniac dans le gaz sortant du réacteur est déterminée par une analyse en ligne par spectrométrie Infra-Rouge à Transformée de Fourrier.

Dans le cas des additifs inhibiteurs de dégradation selon l'invention, la teneur en $NH_3$ est toujours très faible devant celle obtenue dans le cas d'un additif anti-oxygène conventionnel (voir courbe 3), le sulfite de sodium et celle obtenue en absence d'additif inhibiteur de dégradation (voir courbe 2).

Cet exemple montre donc bien que les additifs inhibiteurs de dégradation selon l'invention sont efficaces pour réduire les émissions de composés de dégradation volatils. Par conséquent, dans un procédé industriel utilisant une solution absorbante contenant des additifs inhibiteurs de dégradation selon l'invention, les émissions de composés volatils en tête d'absorbeur seront bien moindres qu'en absence d'additifs inhibiteurs de dégradation.

EXEMPLE 3 :

Cet exemple montre que l'utilisation des additifs inhibiteurs de dégradation de l'amine permet l'augmentation de la concentration en amine sans augmentation de la dégradation. Cet exemple donne les résultats obtenus avec une solution aqueuse de MEA à 40% poids.

Les essais de dégradation d'une amine en solution aqueuse sont effectués selon le mode opératoire suivant.

100g de solution de MEA 40% poids dans l'eau désionisée sont placés dans un réacteur en verre surmonté d'un condenseur pour éviter l'évaporation de l'eau. Le réacteur est chauffé à 80°C dans un bloc chauffant électrique. La solution est agitée à 1000 tours par minute par un barreau aimanté. La présence de contre pales empêche la formation d'un vortex. Un gaz est mis en contact avec la solution à l'aide d'un tube plongeant pendant 7 jours à pression atmosphérique. Les essais sont conduits soit en absence soit en présence d'un additif inhibiteur de dégradation incorporé dans la solution aqueuse d'amine à 0,25% poids.

L'essai est conduit en présence de $CO_2$ et d'oxygène : le gaz mis en contact avec la solution est un mélange

de 7Nl/h d'air atmosphérique, c'est-à-dire de l'air ambiant non purifié, et de 0,033 Nl/h de $CO_2$ réalisé dans une chambre de mélange. Le gaz contient donc du $CO_2$, de l'azote et de l'oxygène, la teneur en oxygène dans le gaz étant de 21% environ.

Une analyse par chromatographie en phase gazeuse de la solution ainsi dégradée est réalisée à la fin de l'essai. La méthode chromatographique utilise une colonne polaire, un gaz vecteur, l'hélium, un étalon interne, le triéthylèneglycol et une détection FID (Flame Induced Detection). Cette analyse permet de déterminer la concentration résiduelle de MEA.

Une vitesse moyenne de dégradation sur la durée de l'essai peut donc être calculée :

$$vitesse_moyenne_de_dégradation = \frac{[MEA]_{initiale} - [MEA]_{finale}}{durée_essai} * masse_de_solution$$

De même un taux de dégradation peut être calculé :

$$taux_de_dégradation = \left(1 - \frac{[MEA]finale}{[MEA]initiale}\right) * 100$$

Le tableau 2 présente les vitesses moyennes de dégradation de la MEA obtenues dans les mêmes conditions dans le cas n°8 d'une MEA 40% poids sans additif et le cas n°2 d'une MEA 30% poids sans additif défini dans l'exemple 1.

Tableau 2 : comparaison des vitesses moyennes de dégradation de la MEA 30% et 40% poids en absence d'un additif inhibiteur de dégradation selon l'invention.

| CAS | Teneur en $O_2$ | [MEA] en % poids | Vitesse moyenne de dégradation (g/jour) |
|---|---|---|---|
| 2 | 21% | 30% | 2,99 |
| 8 | 21% | 40% | 3,72 |

Le tableau 2 confirme bien qu'une solution aqueuse de MEA 40% poids se dégrade plus vite qu'une solution de MEA 30% poids. Ainsi pour une même durée, la masse de MEA dégradée est plus importante dans le cas d'une solution aqueuse de MEA 40% poids.

Le tableau 3 donne les taux de dégradation d'une solution aqueuse de MEA 40% poids, en présence d'un additif inhibiteur de dégradation ou non :

- Cas n°8 : sans additif
- Cas n°9 : en présence d'un additif selon l'invention, le 3,4,5,6-tétrahydro-2-pyrimidinethiol

Tableau 3 : comparaison des taux de dégradation de la MEA 40% poids obtenus dans l'eau à 80°C en absence et en présence d'un additif inhibiteur de dégradation selon l'invention.

| CAS | Teneur en $O_2$ | Nom de l'additif | Taux de dégradation |
|---|---|---|---|
| 8 | 21% | - | 66% |
| 9 | 21% | 3,4,5,6-tétrahydro-2-pyrimidinethiol | <3% |

Il apparaît clairement qu'en présence d'un additif selon l'invention, la dégradation de la MEA à 40 % poids dans l'eau peut être considérée comme nulle car inférieure à l'incertitude de la mesure qui est de 3%.

En conclusion, dans le cas de la MEA, les additifs selon l'invention permettent d'augmenter la concentration en amine communément utilisée par l'homme du métier sans augmenter la dégradation de l'amine.

EXEMPLE 4 :

Cet exemple montre que l'utilisation des additifs inhibiteurs de dégradation de l'amine permet l'augmentation

de la concentration en amine sans augmenter les émissions de produits de dégradation volatils. Cet exemple donne les résultats obtenus avec une solution aqueuse de MEA à 40% poids.

La figure 2 présente un suivi de la concentration en ammoniac dans le gaz sortant du réacteur dans les cas 8 et 9 définis dans l'exemple 3. [A] correspond à la concentration en ammoniac en ppm volume dans le gaz de sortie du réacteur, t représente le temps exprimé en jours. Les cas 8 et 9 sont représentés respectivement par la courbe 8 avec des ronds blancs et la courbe 9 avec des losanges noirs.

La concentration en ammoniac dans le gaz sortant du réacteur est déterminée par une analyse en ligne par spectrométrie Infra-Rouge à Transformée de Fourrier.

Dans le cas de l'additif inhibiteur de dégradation selon l'invention, la teneur en $NH_3$ est inférieure à 10ppm pendant toute la durée du test alors qu'elle dépasse rapidement les 2000ppm sans additif inhibiteur de dégradation. Il apparaît clairement qu'en présence d'un additif selon l'invention, les émissions d'ammoniac liées à la dégradation de la MEA à 40 % poids dans l'eau sont considérablement réduites.

Cet exemple montre donc bien, dans le cas de la MEA, que les additifs selon l'invention permettent d'augmenter la concentration en amine communément utilisée par l'homme du métier sans augmenter les émissions d'ammoniac.

Par conséquent, dans un procédé industriel utilisant une solution absorbante contenant des additifs inhibiteurs de dégradation selon l'invention, les émissions de composés volatils en tête d'absorbeur seront bien moindres qu'en absence d'additifs inhibiteurs de dégradation même si la concentration en amine est augmentée par rapport à la concentration communément utilisée par l'homme du métier.

## Revendications

**1.** Procédé pour absorber des composés acides contenus dans un effluent gazeux, dans lequel on met en contact l'effluent gazeux avec une solution absorbante, ladite solution comportant :

a) au moins une amine,
b) de l'eau,
c) au moins un composé inhibiteur de dégradation pour limiter la dégradation de ladite amine, ledit composé inhibiteur de dégradation répondant à l'une des formules générales suivantes :

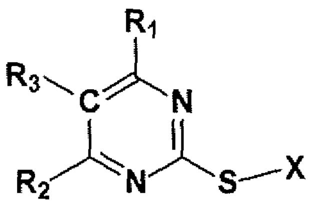

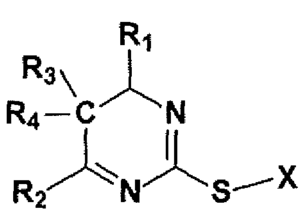

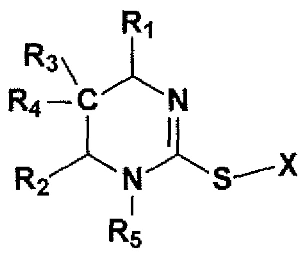

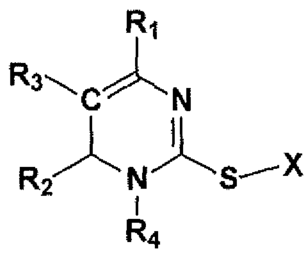

dans lesquelles X est choisi parmi

- o un atome d'hydrogène,
- ◦ un élément alcalin ou alcalino-terreux,
- ◦ un métal monovalent ou multivalent,
- ◦ un cation ammonium $NH_4^+$ ou résultant de la protonation d'une fonction amine,
- ◦ un cation phosphonium,

dans lesquelles chacun des radicaux $R_1$, $R_2$ et $R_3$ est choisi indépendamment parmi :

- un atome d'hydrogène,
- un radical hydrocarboné comprenant 1 à 12 atomes de carbone,
- un radical

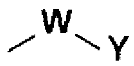

dans lequel W est choisi parmi un atome de soufre et un atome d'oxygène et Y est choisi parmi :

- un atome d'hydrogène,
- un radical hydrocarboné comprenant de 1 à 12 atomes de carbone, ◦ un élément alcalin ou alcalino-terreux,
- un métal monovalent ou multivalent,
- un cation ammonium $NH_4^+$ ou résultant de la protonation d'une fonction amine,
- un cation phosphonium,

et dans lesquelles chacun des radicaux $R_4$ et $R_5$ est choisi indépendamment parmi :

- un atome d'hydrogène,
- un radical hydrocarboné comprenant de 1 à 12 atomes de carbone.

**2.** Procédé selon la revendication 1, dans lequel au moins l'un des radicaux $R_1$, $R_2$, $R_3$, X et Y est un groupe hydrocarboné renfermant entre 1 et 12 atomes de carbone et contient, en outre, au moins un composé choisi parmi un hétéroatome et un halogène.

**3.** Procédé selon l'une des revendications 1 et 2, dans lequel au moins l'un des radicaux $R_1$, $R_2$, $R_3$, X et Y est un groupe hydrocarboné renfermant entre 1 et 12 atomes de carbone et comporte, en outre, au moins une fonction choisie parmi le groupe : une fonction hydroxyle, une fonction cétone, une fonction carboxylique et une fonction nitrile.

**4.** Procédé selon l'une des revendications précédente, dans lequel la solution comporte entre 10% et 99% poids d'amine, entre 1% et 90% poids d'eau et entre 5 ppm et 5% poids de composé inhibiteur de dégradation.

**5.** Procédé selon l'une des revendications précédentes, dans lequel le composé inhibiteur de dégradation est choisi parmi le groupe contenant : le 2-thiouracil (ou 4-hydroxy-2-mercaptopyrimidine), un sel du 2-thiouracil (ou 4-hydroxy-2-mercaptopyrimidine), le 4-thiouracil (ou 2-hydroxy-4-mercaptopyrimidine), un sel du 4-thiouracil (ou 2-hydroxy-4-mercaptopyrimidine), l'acide 2-thiobarbirurique (ou 4,6-dihydroxypyrimidine-2-thiol), un sel de l'acide 2-thiobarbirurique (ou 4,6-dihydroxypyrimidine-2-thiol), la 2-mercaptopyrimidine, un sel de la 2-mercaptopyrimidine, le 3,4,5,6-tétrahydropyrimidine-2-thiol, un sel du 3,4,5,6-tétrahydropyrimidine-2-thiol, le 4,5-dihydro-4,4,6-triméthyl-2-pyrimidinethiol, un sel du 4,5-dihydro-4,4,6-triméthyl-2-pyrimidinethiol, la 4-hydroxy-2-mercapto-6-méthylpyrimidine, un sel de la 4-hydroxy-2-mercapto-6-méthylpyrimidine, la 2-mercapto-4-méthylpyrimidine, un sel de la 2-mercapto-4-méthylpyrimidine, et la 4-méthyl-2-(méthylthio)pyrimidine.

**6.** Procédé selon l'une des revendications précédentes, dans lequel l'amine est choisie parmi le groupe contenant : la N,N,N',N',N"-pentaméthyldiéthylènetriamine, la pipérazine, la monoéthanolamine, la diéthanolamine, la méthyldiéthanolamine, la diisopropanolamine, la diglycolamine, un sel de la glycine et un sel de la taurine.

**7.** Procédé selon l'une des revendications précédentes, dans lequel l'amine est la monoéthanolamine et dans laquelle le composé inhibiteur de dégradation est choisi parmi l'acide 2-thiobarbiturique, le 2-thiouracil, et le 3,4,5,6-tétrahydro-2-pyrimidinethiol.

**8.** Procédé selon l'une des revendications 6 et 7, dans lequel la solution comporte au moins 39% poids de monoéthanolamine.

**9.** Procédé selon l'une des revendications précédentes, dans lequel la solution aqueuse est mise en oeuvre pour absorber des composés acides contenus dans l'un des effluents du groupe contenant le gaz naturel, les fumées de combustion, les gaz de synthèse, les gaz de raffinerie, les gaz obtenus en queue du procédé Claus, les gaz de fermentation de biomasse, les gaz de cimenterie et les fumées d'incinérateur.

**10.** Procédé selon la revendication 9, dans lequel l'effluent gazeux comporte au moins 500 ppm volumique d'oxygène.

## Patentansprüche

**1.** Verfahren zum Absorbieren von Säureverbindungen, die in einem Abgas enthalten sind, wobei das Abgas mit einer Absorptionslösung in Kontakt gebracht wird, wobei die Lösung Folgendes umfasst:

a) mindestens ein Amin,
b) Wasser,
c) mindestens eine den Abbau hemmende Verbindung, um den Abbau des Amins zu begrenzen, wobei die den Abbau hemmende Verbindung einer der folgenden allgemeinen Formeln entspricht:

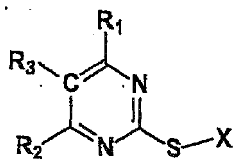

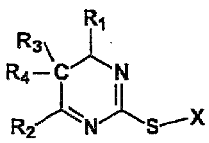

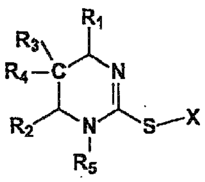

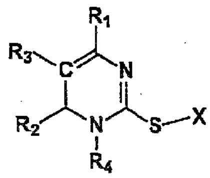

worin X ausgewählt ist aus:

- einem Wasserstoffatom,
- einem alkalischen oder erdalkalischen Element,
- einem einwertigen oder mehrwertigen Metall,
- einem Ammoniumkation $NH_4^+$ oder einem, das aus der Protonierung einer Aminfunktion resultiert,
- einem Phosphoniumkation,

worin jeder der Reste $R_1$, $R_2$ und $R_3$ unabhängig ausgewählt ist aus:

- einem Wasserstoffatom,
- einem Kohlenwasserstoffrest, der 1 bis 12 Kohlenstoffatome aufweist,
- einem Rest

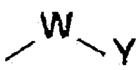
,

worin W ausgewählt ist aus einem Schwefelatom und einem Sauerstoffatom und Y ausgewählt ist aus:
- einem Wasserstoffatom,
- einem Kohlenwasserstoffrest, der 1 bis 12 Kohlenstoffatome aufweist,
- einem alkalischen oder erdalkalischen Element,
- einem einwertigen oder mehrwertigen Metall,
- einem Ammoniumkation $NH_4^+$ oder einem, das aus der Protonierung einer Aminfunktion resultiert,
- einem Phosphoniumkation,

und worin jeder der Reste $R_4$ und $R_5$ unabhängig ausgewählt ist aus:

- einem Wasserstoffatom,
- einem Kohlenwasserstoffrest, der 1 bis 12 Kohlenstoffatome aufweist.

**2.** Verfahren nach Anspruch 1, wobei mindestens einer der Reste $R_1$, $R_2$, $R_3$, X und Y für eine Kohlenwasserstoffgruppe steht, die zwischen 1 und 12 Kohlenstoffatome umfasst und ferner mindestens eine Verbindung enthält, ausgewählt aus einem Heteroatom und einem Halogen.

**3.** Verfahren nach einem der Ansprüche 1 und 2, wobei mindestens einer der Reste $R_1$, $R_2$, $R_3$, X und Y für eine Kohlenwasserstoffgruppe steht, die zwischen 1 und 12 Kohlenstoffatome umfasst und ferner mindestens eine Funktion aufweist, ausgewählt aus der Gruppe: eine Hydroxylfunktion, eine Ketonfunktion, eine Carboxylfunktion und eine Nitrilfunktion.

**4.** Verfahren nach einem der vorhergehenden Ansprüche, wobei die Lösung zwischen 10 Gew.-% und 99 Gew.-% Amin, zwischen 1 % und 90 % Wasser und zwischen 5 ppm und 5 Gew.-% einer den Abbau hemmenden Verbindung aufweist.

**5.** Verfahren nach einem der vorhergehenden Ansprüche, wobei die den Abbau hemmende Verbindung ausgewählt ist aus der Gruppe, enthaltend: 2-Thiouracil (oder 4-Hydroxy-2-mercaptopyrimidin), einem Salz von 2-Thiouracil (oder 4-Hydroxy-2-mercaptopyrimidin), 4-Thiouracil (oder 2-Hydroxy-4-mercaptopyrimidin), einem Salz von 4-Thiouracil (oder 2-Hydroxy-4-mercaptopyrimidin), 2-Thiobarbitursäure (oder 4,6-Dihydroxypyrimidin-2-thiol), einem Salz der 2-Thiobarbitursäure (oder 4,6-Dihydroxypyrimidin-2-thiol), 2-Mercaptopyrimidin, einem Salz von 2-Mercaptopyrimidin, 3,4,5,6-Terahydropyrimidin-2-thiol, einem Salz von 3,4,5,6-Tetrahydropyrimidin-2-thiol, 4,5-Dihydro-4,4,6-trimethyl-2-pyrimidinthiol, einem Salz von 4,5-Dihydro-4,4,6-trimethyl-2-pyrimidinthiol, 4-Hydroxy-2-mercapto-6-methylpyrimidin, einem Salz von 4-Hydroxy-2-mercapto-6-methylpyrimidin, 2-Mercapto-4-methylpyrimidin, einem Salz von 2-Mercapto-4-methylpyrimidin und 4-Methyl-2-(methylthio)pyrimidin.

**6.** Verfahren nach einem der vorhergehenden Ansprüche, wobei das Amin ausgewählt ist aus der Gruppe, enthaltend: N,N,N',N',N"-Pentamethyldiethylentriamin, Piperazin, Monoethanolamin, Diethanolamin, Methyldiethanolamin, Diisopropanolamin, Diglycolamin, einem Glycinsalz und einem Taurinsalz.

**7.** Verfahren nach einem der vorhergehenden Ansprüche, wobei das Amin Monoethanolamin ist, und wobei die den Abbau hemmende Verbindung ausgewählt ist aus 2-Thiobarbitursäure, 2-Thiouracil und 3,4,5,6-Tetrahydro-2-pyrimidinthiol.

**8.** Verfahren nach einem der Ansprüche 6 und 7, wobei die Lösung mindestens 39 Gew.-% Monoethanolamin aufweist.

**9.** Verfahren nach einem der vorhergehenden Ansprüche, wobei die wässrige Lösung eingesetzt wird, um Säureverbindungen zu absorbieren, die in einem der Abflüsse der Gruppe, enthaltend Erdgas, Rauchgase, Synthesegase, Raffineriegase, Gase, die am Ende des Claus-Verfahrens erhalten werden, Biomassefermentationsgase, Gase aus der Zementverarbeitung und Rauchgase aus einer Verbrennungsanlage enthalten sind.

**10.** Verfahren nach Anspruch 9, wobei das Abgas mindestens 500 Vol.-ppm Sauerstoff aufweist.

## Claims

**1.** A method for absorbing acid compounds contained in a gaseous effluent wherein the gaseous effluent is contacted with an absorbent solution, said solution comprising:

a) at least one amine,
b) water,
c) at least one degradation inhibiting compound for limiting the degradation of said amine, said degradation inhibiting compound meeting one of the following general formulas:

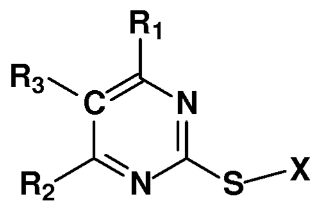

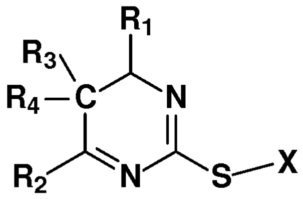
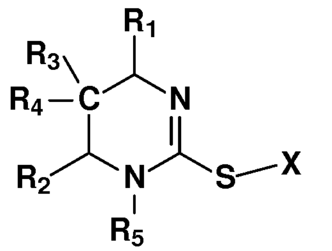
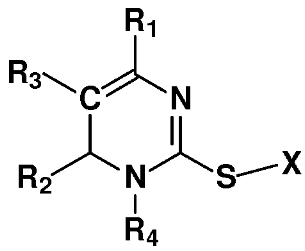

wherein X is selected from among:

- a hydrogen atom,
- an alkaline or alkaline-earth element,
- a monovalent or multivalent metal,
- an ammonium cation $NH_4^+$ or resulting from the protonation of an amine function,
- a phosphonium cation,

wherein each radical $R_1$, $R_2$ and $R_3$ is independently selected from among:

- a hydrogen atom,
- a hydrocarbon radical comprising 1 to 12 carbon atoms,
- a radical

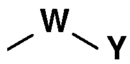

wherein W is selected from among a sulfur atom and an oxygen atom and Y is selected from among:

- a hydrogen atom,
- a hydrocarbon radical comprising 1 to 12 carbon atoms,
- an alkaline or alkaline-earth element,
- a monovalent or multivalent metal,
- an ammonium cation $NH_4^+$ or resulting from the protonation of an amine function,
- a phosphonium cation,

and wherein each radical $R_4$ and $R_5$ is independently selected from among:

- a hydrogen atom,
- a hydrocarbon radical comprising 1 to 12 carbon atoms.

**2.** A method as claimed in claim 1, wherein at least one of radicals $R_1$, $R_2$, $R_3$, X and Y is a hydrocarbon group containing between 1 and 12 carbon atoms and additionally contains at least one compound selected from among a heteroatom and a halogen.

**3.** A method as claimed in any one of claims 1 and 2, wherein at least one of radicals $R_1$, $R_2$, $R_3$, X and Y is a hydrocarbon group containing between 1 and 12 carbon atoms and additionally contains at least one function selected from the group as follows: a hydroxyl function, a ketone function, a carboxylic function and a nitrile function.

**4.** A method as claimed in any one of the previous claims, wherein the solution comprises between 10 wt.% and 99 wt.% amine, between 1 wt.% and 90 wt.% water, and between 5 ppm and 5 wt.% degradation inhibiting compound.

**5.** A method as claimed in any one of the previous claims, wherein the degradation inhibiting compound is selected from the group containing: 2-thiouracil (or 4-hydroxy-2-mercaptopyrimidine), a 2-thiouracil (or 4-hydroxy-2-mercaptopyrimidine) salt, 4-thiouracil (or 2-hydroxy-4-mercaptopyrimidine), a 4-thiouracil (or 2-hydroxy-4-mercaptopyrimidine) salt, 2-thiobarbituric (or 4,6-dihydroxypyrimidine-2-thiol) acid, a 2-thiobarbituric (or 4,6-dihydroxypyrimidine-2-thiol) acid salt, 2-mercaptopyrimidine, a 2-mercaptopyrimidine salt, 3,4,5,6-tetrahydropyrimidine-2-thiol, a 3,4,5,6-tetrahydropyrimidine-2-thiol salt, 4,5-dihydro-4,4,6-trimethyl-2-pyrimidinethiol, a 4,5-dihydro-4,4,6-trimethyl-2-pyrimidinethiol salt, 4-hydroxy-2-mercapto-6-methylpyrimidine, a 4-hydroxy-2-mercapto-6-methylpyrimidine salt, 2-mercapto-4-methylpyrimidine, a 2-mercapto-4-methylpyrimidine salt and 4-methyl-2-(methylthio)pyrimidine.

**6.** A method as claimed in any one of the previous claims, wherein the amine is selected from the group containing: N,N,N',N',N"-pentamethyldiethylenetriamine, piperazine, monoethanolamine, diethanolamine, methyldiethanolamine, diisopropanolamine, diglycolamine, a glycine salt and a taurine salt.

**7.** A method as claimed in any one of the previous claims, wherein the amine is monoethanolamine and wherein the degradation inhibiting compound is selected from among 2-thiobarbituric acid, 2-thiouracil and 3,4,5,6-tetrahydro-2-pyrimidinethiol.

**8.** A method as claimed in any one of claims 6 and 7, wherein the solution comprises at least 39 wt.% monoethanolamine.

**9.** A method as claimed in any one of the previous claims, wherein the aqueous solution is used for absorbing acid compounds contained in one of the effluents of the group consisting of natural gas, combustion fumes, syngas, refinery gas, Claus tail gases, biomass fermentation gas, cement plant gas and incinerator fumes.

**10.** A method as claimed in claim 9, wherein the gaseous effluent comprises at least 500 ppm by volume of oxygen.

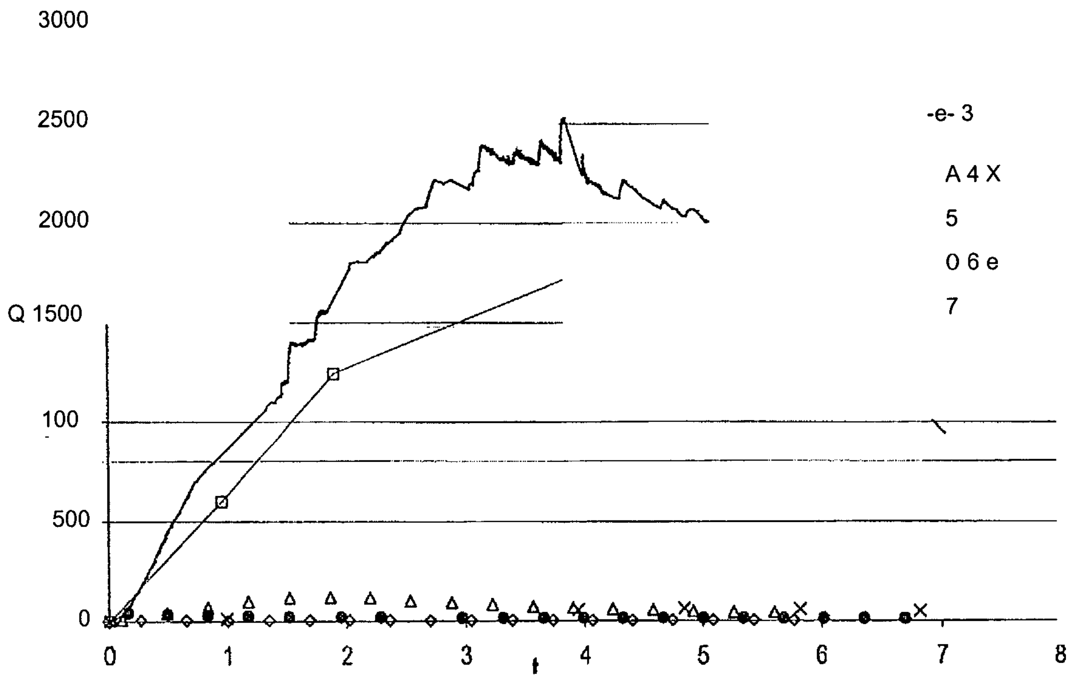
3000
2500
2000
Q 1500
100
500
0
0
1
2
3
t
4
5
6
7
8
-e- 3
A 4 X
5
0 6 e
7

FIG. 1

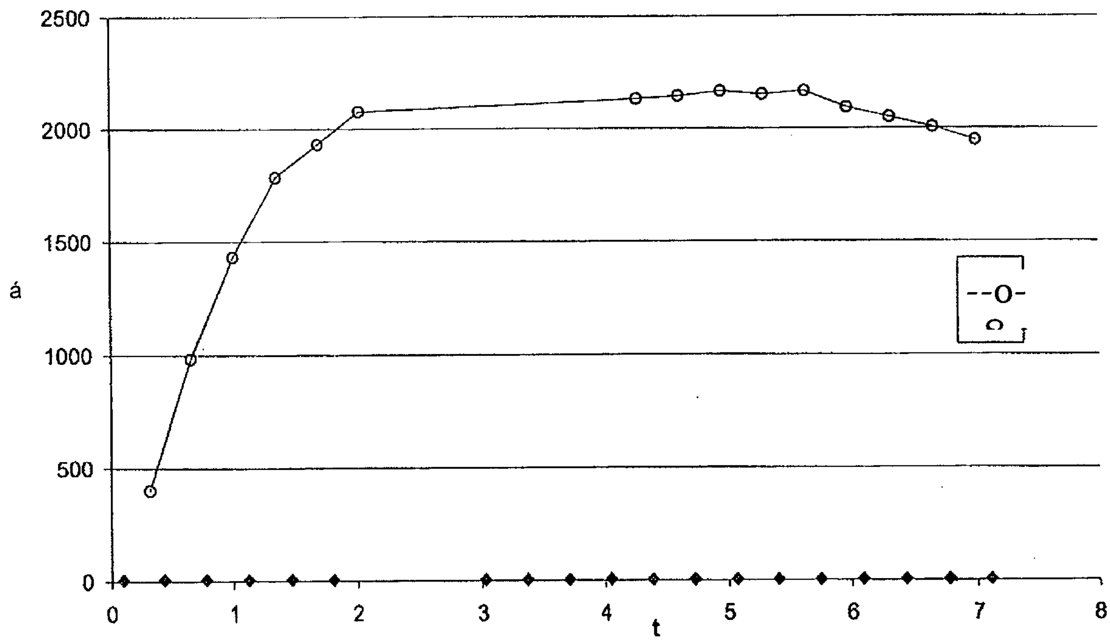
2500
2000
1500
á
1000
500
0
0
1
2
3
4
t
5
6
7
8

FIG. 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2820430
- US 5686016 A
- US 7056482 B
- US 20050202967 A